**Europäisches Patentamt**

**European Patent Office**

**Office européen des brevets**

(11) Publication number: **0 184 942**
**B1**

## (12) EUROPEAN PATENT SPECIFICATION

(45) Date of publication of patent specification: **08.08.90**

(21) Application number: **85309084.3**

(22) Date of filing: **13.12.85**

(51) Int. Cl.⁵: **A 61 K 31/20, A 61 K 47/00**

(54) Improved pharmaceutical composition containing 13-CIS vitamin a acid as the active ingredient.

(30) Priority: **14.12.84 US 681970**

(43) Date of publication of application:
**18.06.86 Bulletin 86/25**

(45) Publication of the grant of the patent:
**08.08.90 Bulletin 90/32**

(84) Designated Contracting States:
**AT BE CH DE FR GB IT LI LU NL**

(56) References cited:
**DE-A-2 838 387**
**US-A-3 124 510**
**US-A-4 322 438**
**US-A-4 464 394**

**CHEMICAL ABSTRACTS, vol. 99, no. 14, 3rd October 1983, page 322,323, abstract no. 110541d, Columbus, Ohio, US; & JP-A-58 41 813 (SUNSTAR, INC.) 11-03-1983**

(73) Proprietor: **ORTHO PHARMACEUTICAL CORPORATION**
**U.S. Route 202 P.O. Box 300**
**Raritan New Jersey 08869-0602 (US)**

(72) Inventor: **Jarosz, Paul J.**
**P.O. Box 133**
**Sergeantsville New Jersey 08557 (US)**

(74) Representative: **Jones, Alan John et al**
**CARPMAELS & RANSFORD 43 Bloomsbury Square**
**London, WC1A 2RA (GB)**

Courier Press, Leamington Spa, England.

**Description**

The present invention relates to pharmaceutical compositions in which 13-cis vitamin A acid is the active ingredient. More particularly, the invention relates to improved compositions in which the bioavailability of the active ingredient is enhanced.

13-Cis vitamin A acid, (also known as 13-cis retinoic acid and isotretinoin) its isomers and some of its analogs are known to have beneficial effects in the treatment of dermatopathic conditions, i.e., diseases of the skin, including acne and the keratinizing skin disorders.

Although 13-cis vitamin A acid is generally less toxic than all-trans vitamin A acid, side effects from their use such as headaches, nausea, vomiting, hair loss, nose bleeds, irritation of the oral and pharyngeal mucosa and liver toxicity due to storage of vitamin A in the liver have been reported. Animal studies conducted with 13-cis vitamin A acid suggest that teratogenic effects may occur. In addition, there have been multiple reports of birth defects and spontaneous abortions in humans following the first trimester exposure to 13-cis vitamin A acid.

U.S. Patent No. 4,464,394 discloses compositions and methods for using 13-cis vitamin A acid. However, only a general description of the compositions is given and no data on the bioavailability of the active ingredient in the composition are disclosed.

13-Cis vitamin A acid is a relatively water insoluble compound that degrades when exposed to light and atmospheric oxygen. Because of its instability and relative insolubility, the bioavailability of the drug in the blood stream after oral administration of compositions wherein the drug is the active ingredient has always been in question.

Because of the suspected low bioavailability of the compound and the adverse side effects attendant to its use, it would be desirable to provide a dosage form in which the drug is stable and predictably bioavailable. In this way, the desired therapeutic effect can be achieved by the administration of the minimum amounts of drug required for successful treatment of a given disorder. This would result in a potential reduction in the incidence and severity of the known adverse reactions associated with its uses, since the adverse reactions could be amplified by unpredictable bioavailability.

It is an object of this invention to provide pharmaceutical compositions containing 13-cis vitamin A acid as the active ingredient in which the active ingredient is stable and more bioavailable than in previously available compositions. It is another object of this invention to provide pharmaceutical compositions containing 13-cis vitamin A acid as the active ingredient in which the vitamin A acid compound is approximately two times more physiologically available than in presently available compositions. These and other objects of the invention will be apparent to one skilled in the art from the detailed description given hereinafter.

13-Cis vitamin A acid can be administered enterally or parenterally in the form of a tablet, capsule, dragee, syrup, suspension, solution or suppository. A preferred dosage form for oral administration is a capsule of gelatin, methyl cellulose or other suitable material which will dissolve readily, releasing its contents in the digestive tract.

In accordance with the present invention, the improved pharmaceutical composition is comprised of a suspending agent, an antioxidant to stabilize the active ingredient, a chelating agent, a vehicle and the active ingredient. The suspending agent is present in the composition in a ratio between 3 and 22%; the antioxidant is present in a ratio between 0.001 and 0.5%; the chelating agent is present in a ratio between 0.01 and 0.3%; the active ingredient is present in a ratio between 3 and 12% by weight; and the remainder of the composition is comprised of the vehicle or pharmaceutical carrier which may be present in the range of 65—94%. As the suspending agent, a standard wax mixture** [**J. P. Stanley, The Theory and Practice of Industrial Pharmacy, L. Lackman, H. A. Lieberman & J. L. Kanig, eds.; 2nd ed; Lea & Febiger, Phila. (1976) p. 412).] comprised of 1 part yellow wax, NF, 4 parts hydrogenated vegetable oil, and 4 parts vegetable shortening is employed (hereinafter referred to as the wax mixture). In addition to the standard wax mixture beeswax, paraffin wax and glyceryl-monostearate may also be employed. As the antioxidant, α-tocopherol, butylated hydroxyanisole (BHA), butylated hydroxytoluene (BHT), ascorbyl palmitate and propyl gallate may be employed. Suitable vehicles which can be employed in the formulation include peanut oil, soybean oil, sesame oil, mineral oil, cottonseed oil and polyethylene glycol 400. Any suitable chelating agent, such as, for example, disodium edetate and calcium disodium edetate, may be employed in the composition. In addition to the main components of the composition, it may be desirable in some instances to include a surfactant as one of the components in either the suspending agent or vehicle. The inclusion of a surface active agent has the dual benefit of helping to maintain the active ingredient in uniform suspension in the wax mixture while enhancing the bioavailability of the active ingredient in the blood stream. Suitable surfactants which can be employed include, lecithin, sorbitan monoesters such as sorbitan monoleate, sorbitan monolaurate, sorbitan monopalmitate, sorbitan monostearate; polysorbates prepared from lauric, palmitic, stearic and oleic acids; monononylphenyl ethers of polyethylenglycols such as the nonoxynols; polyoxyethylene monoesters such as polyoxyethylene monostearate, polyoxyethylene monolaurate, polyoxyethylene monooleate, dioctyl sodium sulfosuccinate, sodium laurylsulfate and poloxamers having a molecular weight between 2000 and 8000.

In the formulations of this invention, the 13-cis vitamin A acid may be employed in the form of the free

2

acid or its pharmaceutically acceptable salts such as, for example, the alkali metal salts and the sodium salt in particular.

In a preferred embodiment of the invention, the particle size of the 13-cis vitamin A acid is reduced prior to its incorporation into the formulation. Satisfactory results are obtained from material having a median particle size of less than 12 μm with a decade ratio of less than 25. The preferred median particle size is less than 10 μm with a decade ratio of less than 15. The amount of wax employed is a critical feature of the formulation.

Formulations having a wax content higher than 22% tend to diminish the bioavailability of the active ingredient.

The daily dosage of 13-cis vitamin A acid employed in accordance with the present invention will vary depending upon the severity of the condition being treated. Generally a daily dose by enteral administration of from about 0.5 to 1.0 mg per kg is employed.

Two investigations were conducted in order to determine the bioavailability of 13-cis vitamin A acid in the formulation of this invention. In each investigation comparisons of absorption were evaluated using the analysis of blood samples obtained from subjects following oral administration of the formulation of this invention compared with blood levels obtained from the analysis of samples obtained from subjects following oral administration of various commercially available formulations of the type disclosed in Example 3 of U.S. Patent No. 4,464,394 sold by Hoffman LaRoche under the trademark Accutane, a soft gelatin capsule containing 13-cis Vitamin A acid as the active ingredient. The evaluations were carried out using 5, 10, 20 and 40 mg samples of the formulation of this invention (ORF 16688), 10 mg and 40 mg samples of the commercial preparation (Accutane) and a liquid reference comprised of 20 mg of 13-cis vitamin A acid in 10 ml of alcohol (USP).

The first investigation was divided into two, 3-way crossover studies, Study I and Study II. Thirty, normal, male subjects were enrolled in each segment or study. Whole blood samples were collected at selected times during the 36 hour period following each treatment. Plasma samples were assayed for 13-cis vitamin A acid by a sensitive and specific HPLC method. The area under the plasma 13-cis vitamin A acid concentration $vs$ time curve (AUC), the maximum plasma concentration ($C_{max}$), and the time of maximum concentration ($T_{peak}$) were determined for each subject after each treatment.

Study I compared a 20 mg liquid reference dose, a 4×5 mg capsule dose (ORF 16688), and a 4×10 mg dose of the commercially available formulation (Accutane). The resultant mean values of AUC, $C_{max}$, and $T_{peak}$ are 2777 h ng/ml, 339 ng/ml, and 2 I h, respectively, for the liquid reference; 3418 h ng/ml, 399 ng/ml, and 2.1 h, respectively, for the ORF 16688 4×5 mg capsule; and 2814 h ng/ml, 306 ng/ml, and 2.7 h, respectively, for the commercially available 4×10 mg capsule. An ANOVA showed no differences in the $T_{peak}$ values of the three formulations and no differences in the AUC or $C_{max}$ values for the liquid reference and the commercially available formulation. The AUC and $C_{max}$ values for these two latter formulations are, however, statistically significantly less than the AUC or $C_{max}$ values of the ORF 16688 formulation. The results of Study I indicate that 13-cis vitamin A acid is more than 100% bioavailable from the ORF 16688 5 mg formulation relative to the liquid, that 13-cis vitamin A acid is approximately 50% bioavailable from the commercially available 10 mg formulation relative to the liquid reference, and that 13-cis vitamin A acid is at least 2-fold more available from the ORF 16688 5 mg capsule than from the commercially available 10 mg capsule. The results of this study are shown in Table 1.

TABLE 1

AUC and $C_{max}$ ratios for ORF 16688 capsules versus the commercially available capsule, Accutane

Study I

| Dose | AUC$_{0-36}$ (h. ng/ml) | Ratio* (AUC) | C$_{max}$ (nq/ml) | Ratio* (C$_{max}$) |
|---|---|---|---|---|
| 20 mg liquid reference | 2777 | | 339 | |
| 4×5 mg (ORF 16688) | 3418 | 1.21 | 399 | 1.30 |
| 4×10 mg (Accutane) | 2814 | | 306 | |

*Ratio=ORF 16688/Accutane

In Study II, the second part of the investigation, a comparison of the same 20 mg liquid reference, a 2×10 mg ORF 16688 capsule dose, and a 20 mg ORF 16688 capsule dose was made. This comparison was made, in part, to demonstrate the bioequivalence of ORF 16688 capsules formulated at different times and using different sources of pure drug. The resultant mean AUC, $C_{max}$, and $T_{peak}$ values are 3090 h ng/ml, 373 ng/ml, and 2.5 h for the liquid reference, 3072 h ng/ml, 326 ng/ml, and 3.1 h for the ORF 16688 2×10 capsule; and 3170 h ng/ml, 339 ng/ml, and 3.1 h for the ORF 16688 20 mg capsule. An ANOVA showed that

3

there are no statistically significant differences between the mean AUC, $C_{max}$, and $T_{peak}$ values for the three formulations and suggests that the ORF 16688 10 mg and 20 mg formulations are bioequivalent and afford 100% bioavailability of 13-cis vitamin A acid relative to the liquid reference. The overall results of the studies I and II of the three ORF 16688 formulations relative to a liquid reference are summarized in Table 2. The results indicate that ORF 16688 when administered in a total dose of 20 mg as various strength formulations exhibits uniform bioavailability relative to the liquid reference.

TABLE 2

The bioavailability of three ORF 16688 13-cis vitamin A
acid formulations relative to a liquid reference

| Formulation | No. of subjects | AUC | AUC ratio* | $C_{max}$ | $C_{max}$ ratio |
|---|---|---|---|---|---|
| Liquid reference | 40 | 3090 | 1.00 | 373 | 1.00 |
| ORF 16688 4×5 mg | 27 | 3418 | 1.11 | 399 | 1.07 |
| ORF 16688 2×10 mg | | 3072 | 0.99 | 326 | 0.87 |
| ORF 16688 20 mg | 22 | 3170 | 1.03 | 339 | 0.91 |

*Ratio=Formulation/Liquid Reference

The second investigation was divided into two 2-way cross-over studies and designed to evaluate the ORF 16688 10 and 40 mg dosage forms and to compare them to previously examined dosage forms of ORF 16688 and the commercially available formulation (Accutane). The bioequivalence of 13-cis vitamin A acid in ORF 16688 10 and 40 mg capsules and 10 and 40 mg capsules of the commercially available formulations (Accutane) was determined as follows:

Two separate open-label, two-way crossover studies were conducted in 60 normal male subjects in order to evaluate the bioequivalence of 13-cis vitamin A acid, ORF 16688, 10 and 40 mg capsules with respect to 10 and 40 mg capsules of the commercially available product. One study compared 10 mg capsules of the two formulations administered as a single oral dose of 20 mg of 13-cis vitamin A acid (i.e., 2×10 mg capsules), and the other study compared 40 mg capsules given as a single oral dose of 40 mg (i.e., 1×40 mg capsule). For each of the two studies 30 healthy, male volunteers were randomly assigned to two subgroups of 15, and given the capsules orally. Following a two week washout period, the subgroups within a study were crossed over.

Plasma 13-cis vitamin A acid concentrations were measured at specified times over a 36 hour period after oral administration, using a high performance liquid chromatographic method. As described above, three bioavailability parameters, AUC, $C_{max}U$, and $T_{peak}$ were determined. The mean values (±S.D.) of AUC, $C_{max}$, and $T_{peak}$ were 3387±841 h ng/ml, 388±128 ng/ml, and 2.9±1.0 h, respectively, for 2×10 mg capsules (ORF 16688) and 2306±846 h ng/ml, 252±93 ng/ml, and 2.7±0.9 h, respectively for the 2×10 mg capsules (commercial product). For the 40 mg capsules, the mean values (±S.D.) of AUC, $C_{max}U$, and $T_{peak}$ were 5877±2271 h ng/ml, 737±347 ng/ml, and 2.5±0.9 h, respectively, for the ORF 16688 formulation and 3649±1687 h ng/ml, 377±209 ng/ml, and 3.1±1.1 h for the commercial product. The coefficients of variation for all three bioavailability parameters obtained with ORF 16688 capsules were consistently smaller than those obtained with the commercial capsules. The statistical evaluation of these data showed that the mean values of AUC and $C_{max}U$ obtained with ORF 16688 10 and 40 mg capsules were significantly higher than those obtained with the commercial product 10 and 40 mg capsules. The means values of the time of occurrence of the peak ($T_{peak}$) were not different for the 2×10 mg capsules but were statistically significantly different for the 40 mg dosage forms with the commercially available form having a prolonged absorption.

The results clearly indicate that ORF 16688 capsules provide increased extent of absorption with less variable plasma concentrations, and that the bioavailability of 13-cis vitamin A acid from ORF 16688 2×10 mg capsules was approximately 1.6 times that form a corresponding dose of the commercial product (2×10 mg) and was equivalent to the bioavailability of the 40 mg capsules of the commercially available product. In addition, the data indicate that the bioavailability of 13-cis vitamin A acid from a 40 mg ORF 16688 capsule is twice as much as that from the corresponding 40 mg commercial available capsule. The results of the study are summarized in Table 3.

TABLE 3

AUC and $C_{max}$ ratios for ORF 16688 capsules versus the
commercially available capsule, Accutane

| Dose | $AUC_{0-36}$ (h. ng/ml) | Ratio* (AUC) | $C_{max}$ (ng/ml) | Ratio* ($C_{max}$) |
|---|---|---|---|---|
| 1×40 mg (ORF 16688) | 5877±2271 | 1.91±0.87 | 737±347 | 2.35±1.22 |
| 1×40 mg (Accutane) | 3649±1687 | | 377±209 | |
| 2×10 mg (ORF 16688) | 3387±841 | 1.58±0.51 | 388±128 | 1.66±0.66 |
| 2×10 mg (Accutane) | 2306±846 | | 252±93 | |

*Ratio=ORF 16688/Accutane

The following examples are illustrative of the present invention but are not intended to limit the scope of the invention as defined by the appended claims:

Example 1
Soft gelatin capsules were filled with the following composition:

| Ingredient | mg/capsule |
|---|---|
| 13-cis vitamin A acid | 5.00 |
| Wax mixture | 34.6 |
| BHA (NF) | 0.110 |
| Disodium Edetate (USP) | 0.495 |
| Soybean oil (USP) | 124.8 |
| | 165.0 mg |

Example 2
Soft gelatin capsules were filled with the following compositions:

| Ingredient | mg/capsules |
|---|---|
| 13-Cis vitamin A acid | 10.00 |
| Wax mixture | 32.50 |
| BHA (NF) | 0.110 |
| Disodium Edetate (USP) | 0.495 |
| Soybean oil (USP) | 121.9 |
| | 165.0 mg |

Example 3
Soft gelatin capsules were filled with the following composition:

| Ingredient | mg/capsule |
|---|---|
| 13-cis vitamin A acid | 20.00 |
| Wax mixture | 65.00 |
| BHA (NF) | 0.220 |
| Disodium Edetate (USP) | 0.990 |
| Soybean oil (USP) | 243.8 |
| | 330.0 mg |

Example 4
Soft gelatin capsules were filled with the following composition:

| Ingredient | mg/capsule |
|---|---|
| 13-cis vitamin A acid | 40.00 |
| Wax mixture | 45.00 |
| BHA (NF) | 0.22 |
| Disodium Edetate (USP) | 0.99 |
| Soybean oil (USP) | 243.8 |
| | 330.01 mg |

Example 5
Soft gelatin capsules were filled with the following composition:

| Ingredient | mg/capsule |
|---|---|
| 13-cis vitamin A acid | 5.00 |
| Beeswax | 34.6 |
| Propyl gallate (NF) | 0.110 |
| Disodium Edetate (USP) | 0.495 |
| Soybean oil (USP) | 124.8 |
| | 165.0 mg |

Example 6
Soft gelatin capsules were filled with the following composition:

| Ingredient | mg/capsule |
|---|---|
| 13-cis vitamin A acid | 20.00 |
| Lecithin | 9.90 |
| Beeswax | 16.5 |
| BHA (NF) | 0.220 |
| Disodium Edetate (USP) | 0.990 |
| Cottonseed oil (USP) | 282.4 |
| | 330.0 mg |

The effectiveness of the formulation
The safety and efficacy of the ORF 16688 formulation was determined in a double blind comparative study against a placebo in the treatment of patients with severe papulopustular and or cystic acne. The formulation was administered orally in 2 daily doses at 0.5—1.0 mg/kg/day compared with a placebo capsule. Forty healthy patients were enrolled in the study. Twenty of the patients were randomly assigned the placebo capsules. Ten (10) mg capsules of ORF 16688 and placebo were supplied. The duration of drug administration was 20 weeks or less if a greater than 70% reduction of cysts or other inflammatory lesions was observed. Of the 20 patients who were on the active medication, 14 completed the study. The results of the study are summarized in Table 3.

TABLE 4

| Investigator's clinical evaluation/ global evaluation at D/C* | Number of patients |
| --- | --- |
| Excellent/None | 4 |
| Excellent/Mild | 4 |
| Good/Mild | 6 |

| Investigator's clinical evaluation/ global evaluation at follow-up | Number of patients |
| --- | --- |
| Excellent/None | 9 |
| Excellent/Mild | 2 |
| Good/Mild | 3 |

*Discontinuation

## Claims

1. A pharmaceutical composition useful in the oral treatment of dermatopathies comprising 3% to 22% by weight of a suspending agent, 0.001% to 0.5% by weight of an antioxidant, 0.01% to 0.3% by weight of a chelating agent, 3% to 12% by weight of 13-cis vitamin A acid or a pharmaceutically acceptable salt thereof, and 65% to 94% by weight of a pharmaceutical carrier.

2. The composition of claim 1 wherein the suspending agent is a wax mixture, beeswax, paraffin or glyceryl-monostearate.

3. The composition of claim 1 or claim 2 wherein the antioxidant is butylated hydroxyanisol, butylated hydroxytoluene, propyl gallate, α-tocopherol or ascorbyl palmitate.

4. The composition of any one or claims 1 to 3 wherein the chelating agent is disodium edetate or calcium disodium edetate.

5. The composition of any of claims 1 to 4 wherein the pharmaceutical carrier is soybean oil, peanut oil, sesame oil, mineral oil, cottonseed oil or polyethylene glycol 400.

6. The pharmaceutical composition of claim 1 comprising 3% to 22% by weight wax, 0.001% to 0.5% by weight of butylated hydroxyanisol, 0.01% to 0.3% by weight of disodium edetate, 65% to 94% by weight of soybean oil and 3% to 12% by weight 13-cis vitamin A acid.

7. The pharmaceutical composition of claim 1 comprising 21% wax mixture, 0.67% butylated hydroxyanisole 0.30% disodium edetate, 75.6% soybean oil and 3% 13-cis vitamin A acid.

8. The composition of any one of claims 1 to 7, in unit dosage form as a soft gelatin capsule.

9. The composition of any one of claims 1 to 8 wherein a surfactant is additionally present in the suspending agent.

10. The composition of any one of claims 1 to 9 wherein a surfactant is additionally present in the carrier.

11. The composition of claim 9 or claim 10 wherein the surfactant is a lecithin, sorbitan monoester, polysorbate, poloxamer, polyoxyethylene monoester, polyoxyethylene lauryl ether, dioctylsodium sulfosuccinate, monon.nonylphenyl ether of a polyethyleneglycol or sodium laurylsulfate.

12. A composition according to any one of claims 1 to 11 for use in the treatment of dermatopathies.

13. A method of preparing a composition according to any one of claims 1 to 11 comprising intimately admixing the components of the composition.

## Patentansprüche

1. Pharmazeutische Zusammensetzung, die sich zur oralen Behandlung von Dermatopathein eignet, umfassend 3 bis 22 Gew.-% eines Suspendiermittels, 0,001 bis 0,5 Gew.-% eines Antioxidationsmittels, 0,01 bis 0,3 Gew.-% eines Chelatisierungsmittels, 3 bis 12 Gew.-% 13-cis-Vitamin A-Säure oder eines pharmazeutisch annehmbaren Salzes hievon und 65 bis 94 Gew.-% eines pharmazeutischen Trägers.

2. Zusammensetzung nach Anspruch 1, worin das Suspendiermittel eine Wachsmischung, Bienenwachs, Paraffin oder Glyceryl-monostearat ist.

3. Zusammensetzung nach Anspruch 1 oder 2, worin das Antioxidationsmittel butyliertes Hydroxyanisol, butyliertes Hydroxytoluol, Propylgallat, alpha-Tocopherol oder Ascorbylpalmitat ist.

4. Zusammensetzung nach einem der Ansprüche 1 bis 3, worin das Chelatisierungsmittel Dinatriumedeteat oder Calciumdinatriumedeteat ist.

7

5. Zusammensetzung nach einem der Ansprüche 1 bis 4, worin der pharmazeutische Träger Sojabohnenöl, Erdnußöl, Sesamöl, Mineralöl, Baumwollsamenöl oder Polyethylenglycol 400 ist.

6. Pharmazeutische Zusammensetzung nach Anspruch 1, umfassend 3 bis 22 Gew.-% Wachs, 0,001 bis 0,5 Gew.-% butyliertes Hydroxyanisol, 0,01 bis 0,3 Gew.-% Dinatriumedeteat, 65 bis 94 Gew.-% Sojabohnenöl und 3 bis 12 Gew.-% 13-cis-Vitamin A-säure.

7. Pharmazeutische Zusammensetzung nach Anspruch 1, umfassend 21% Wachsmischung, 0,67% butyliertes Hydroxyanisol, 0,30% Dinatriumedeteat, 75,6% Sojabohnenöl und 3% 13-cis-Vitamin A-säure.

8. Zusammensetzung nach einem der Ansprüche 1 bis 7 in Dosiereinheitsform als eine Weichgelatinekapsel.

9. Zusammensetzung nach einem der Ansprüche 1 bis 8, worin zusätzlich im Suspendiermittel ein oberflächenaktives Mittel vorliegt.

10. Zusammensetzung nach einem der Ansprüche 1 bis 9, worin zusätzlich im Träger ein oberflächenaktives Mittel vorliegt.

11. Zusammensetzung nach Anspruch 9 oder 10, worin das oberflächenaktive Mittel ein Lecithin, Sorbitanmonoester, Polysorbat, Poloxamer, Polyoxyethylenmonoester, Polyoxylenlaurylether, Dioctylnatriumsulfosuccinat, Monononylphenylether eines Polyethylenglycols oder Natriumlaurylsulfat ist.

12. Zusammensetzung nach einem der Ansprüche 1 bis 11 zur Anwendung in der Behandlung von Dermatopathien.

13. Verfahren zur Herstellung einer Zusammensetzung nach einem der Ansprüche 1 bis 11, umfassend ein inniges Zusammenmischen der Komponenten der Zusammensetzung.

**Revendications**

1. Composition pharmaceutique utile dans le traitement oral des dermatopathies comprenant 3% à 22% en poids d'un agent de suspension, 0,001% à 0,5% en poids d'un antioxydant, 0,01% à 0,3% en poids d'un agent complexant, 3% à 12% en poids d'acide 13-cis vitaminique A ou son sel acceptable en pharmacie et 65% à 94% en poids d'un véhicule pharmaceutique.

2. Composition de la revendication 1, où l'agent de suspension est un mélange de cires, de la cire d'abeille, de la paraffine ou du monostéarate de glycéryle.

3. Composition de la revendication 1 ou de la revendication 2, où l'antioxydant est l'hydroxyanisole butylé, l'hydroxytoluène butylé, le gallate de propyle, l'α-tocophérol ou le palmitate d'ascorbyle.

4. Composition selon l'une des revendications 1 à 3, où l'agent complexant est l'édétate disodique ou l'édétate disodique de calcium.

5. Composition selon l'une quelconque des revendications 1 à 4, où le véhicule pharmaceutique est l'huile de soja, l'huile d'arachide, l'huile de sésame, l'huile minérale, l'huile de coton ou le polyéthylène glycol 400.

6. Composition pharmaceutique selon la revendication 1, comprenant 3% à 22% en poids de cire, 0,001% à 0,5% en poids d'hydroxyanisole butylé, 0,01% à 0,3% en poids d'édétate disodique, 65% à 94% en poids d'huile de soja et 3% à 12% en poids d'acide 13-cis vitaminique A.

7. Composition pharmaceutique de la revendication 1, comprenant 21% du mélange de cires, 0,67% d'hydroxyanisole butylé, 0,30% d'édétate disodique, 75,6% d'huile de soja et 3% d'acide 13-cis vitaminique A.

8. Composition selon l'une quelconque des revendications 1 à 7 sous forme de dose unitaire en une capsule de gélatine molle.

9. Composition selon l'une quelconque des revendications 1 à 8, où un agent tensio-actif est additionnellement présent dans l'agent de suspension.

10. A composition selon l'une quelconque des revendications 1 à 9 où un agent tensio-actif est additionnellement présent dans le véhicule.

11. Composition selon le revendication 9 ou la revendication 10 où l'agent tensio-actif est une lécithine, un monoester de sorbitan, un polysorbate, un poloxamère, un monoester de polyoxyéthylène, le lauryl éther de polyoxyéthylène, le sulfosuccinate de dioctylsodium, le mononynylphényl éther d'un polyéthylène glycol ou le laurylsulfate de sodium.

12. Composition selon l'une quelconque des revendications 1 à 11, à utiliser dans le traitement des dermatopathies.

13. Méthode de préparation d'une composition selon l'une quelconque des revendications 1 à 11, comprenant le mélange intime des composants de la composition.